## Europäisches Patentamt

## European Patent Office

## Office européen des brevets

(19)

(11) Veröffentlichungsnummer: **0 135 051**
**B1**

(12) **EUROPÄISCHE PATENTSCHRIFT**

(45) Veröffentlichungstag der Patentschrift:
**25.01.89**

(51) Int. Cl.⁴: **G 01 N 33/53**, G 01 N 33/533

(21) Anmeldenummer: **84108635.8**

(22) Anmeldetag: **21.07.84**

(54) Verbesserter Immunoassay zur Bestimmung von Antikörpern gegen native-DNA.

(30) Priorität: **25.07.83 US 516583**

(43) Veröffentlichungstag der Anmeldung:
**27.03.85 Patentblatt 85/13**

(45) Bekanntmachung des Hinweises auf die Patenterteilung:
**25.01.89 Patentblatt 89/4**

(84) Benannte Vertragsstaaten:
**AT BE CH DE FR GB IT LI LU NL SE**

(56) Entgegenhaltungen:
**US-A-4 234 563**
**US-A-4 251 514**

(73) Patentinhaber: **WHITTAKER CORPORATION, 10880 Wilshire Boulevard, Los Angeles, California 90024 (US)**

(72) Erfinder: **Burge, Boyce W., 433 Kingsley Ave., Palo Alto, CA 94301 (US)**
Erfinder: **Gibson, Ursula Elisabeth Martha, 171 Sadbury Drive, Milpitas, Ca 95035 (US)**

(74) Vertreter: **Hansmann, Axel, Patentanwälte HANSMANN & VOGESER Albert- Rosshaupter-Strasse 65, D-8000 München 70 (DE)**

EP 0 135 051 B1

# Beschreibung

Gegenstand dieser Erfindung sind verbesserte Immunoassays sowohl zur quantitativen als auch zur qualitativen Bestimmung von Antikörpern, die gegen native DNA gerichtet sind.

Gegen DNA gerichtete Antikörper sind das Resultat einer fehlerhaften Immunantwort gegen verschiedene Zellbestandteile ohne Organ- oder Speziesspezifität. Obwohl diese Antikörper in geringer Anzahl im Serum jedes Menschen auftreten, nimmt die Anzahl im Alter und bei Menschen, die an verschiedenen systemisch rheumatischen oder Autoimmunkrankheiten leiden, beispielsweise an systemischer Lupus, zu.

Verschiedene Methoden sind bekannt, um Anti-DNA-Antikörper in einer Serumprobe nachzuweisen. So wird in der Druckschrift US - A 4.234.563 ein Verfahren zur Bestimmung von gegen native DNA gerichteten Antikörper offenbart. Es wird ein unlösliches Konjugat aus einem Rinderserumalbumin-Derivat und nativer DNA mit dem Serum eines an einer Autoimmun-Krankheit leidenden Patienten inkubiert und dieses mit beispielsweise mit Fluoreszein markierten Anti-Immunoglobulin-Antikörpern versetzt. Die Bestimmung der Anti-DNA-Antikörper erfolgt dann durch Messung der Fluoreszenz. Gemäß der US - A 4.251.514 läßt sich ein entsprechendes Verfahren auch zur Bestimmung von gegen Immunoglobulin G gerichteten Antikörpern im Patientenserum anwenden.

Andere Verfahren beinhalten die Immobilisierung von beispielsweise Thymus-DNA auf einem Zellulose-Filter. Thymus-DNA ist jedoch eine lineare, heterogene DNA und kann kurze, einsträngige Abschnitte enthalten.

Als Nachweistechnik wird im klinischen Labor die indirekte Immunofluoreszenzmethode bevorzugt. Ihr Hauptnachteil besteht jedoch darin, daß die Intensität der Signale subjektiv interpretiert werden muß.

Ein weiteres Problem bei den Anti-Thymus-DNA Assays besteht in der starken Hintergrundfluoreszenz, die durch nichtspezifische Bindung der Serum-Proteine und Immunoglobuline zum Zellulose-Substrat verursacht wird. Die Intensität dieser Hintergrundfluoreszenz variiert von Serum zu Serum und steigt rapide mit den Probenmengen an.

Als zusätzliche Schwierigkeit bei den bekannten Assays hat sich herausgestellt, daß sie entweder ausschließlich objektiv oder subjektiv zu verwenden sind. Das bedeutet, entweder wird die Intensität der Signal-Muster subjektiv durch mikroskopische Untersuchung interpretiert oder durch instrumentelle Bestimmung gemessen; die Fluoreszenz-Mikroskopie sagt jedoch nichts über die Art der Fluoreszenz und die damit verbundene Krankheit aus.

Es wurde nun vermutet, daß bei der Verwendung homogener, ausschließlich doppelsträngiger DNA die Schwierigkeiten mit den bekannten Assays überwunden werden könnten.

Aufgabe der Erfindung war es daher, einen Anti-DNA-Antikörper-Assay zu entwickeln, in dem das immobilisierte Antigen homogen ist und ausschließlich aus doppelsträngiger DNA besteht.

Eine weitere Aufgabe bestand darin, einen Anti-DNA-Antikörper Assay zu entwickeln, der die Hintergrund-Fluoreszenz, die durch nichtspezifische Bindungen der Serum-Proteine und Immunoglobuline der Test-Probe verursacht wird, zu minimieren. Außerdem war es Aufgabe der Erfindung, einen Assay zu entwickeln, der sowohl objektiv als auch subjektiv interpretiert werden kann, so daß der Assay zur schnellen, klinischen Untersuchung eingesetzt werden kann, die zur Zeit schon die Möglichkeit zur Detektierung niedriger Konzentrationen an Anti-DNA-Antikörpern durch mikroskopische Bestimmung bieten. Die vorliegende Erfindung beschreibt eine Methode zur Fluoroimmunobestimmung der Anti-DNA-Antikörper, dadurch gekennzeichnet, daß ein Antigen-Reagenz durch Aufbringung einer im wesentlichen reinen network DNA aus Kinetoplasten auf ein festes Substrat hergestellt wird, daß das Antigen-Reagenz in einer Serumprobe aus einem Wirt, von dem angenommen wird, daß er Antikörper gegen native DNA bildet, komplexiert wird, daß der Antigen -Reagenz-Komplex in einer Lösung markierter Rezeptoren, die in der Lage sind, die Antikörper zu erkennen und zu binden, inkubiert wird und daß die Anwesenheit eines markierten Rezeptors, der an den Antigen-Antikörper-Komplex gebunden ist, nachgewiesen wird.

Die vorliegende Erfindung ist eine bedeutende Verbesserung der bekanntesten Assays, die für den Nachweis von Antikörpern gegen native DNA gebräuchlich sind. Der network-DNA-Immunoassay eliminiert die unerwünschten Eigenschaften der bekannten Assays.

Die network -DNA, die als Antigen im Antigen-Reagenz benutzt wird, stammt aus Kinetoplasten, die man aus Crithidia lucilia oder Crithidia fasiculata gewinnt. Diese network -DNA ist gekennzeichnet durch eine Anzahl individueller, doppelsträngiger DNA-Ringe von jeweils ca. 1 x $10^6$ Daltons. Es gibt ungefähr 10.000 Ringe pro network -DNA und da kreisförmige DNA schon durch Definition doppelsträngig ist, ist das Auftreten kurzer, einzelsträngiger Abschnitte minimiert.

Die gereinigte Crithidia-network -DNA wird auf aluminisierter $Mylar^R$-Oberfläche fixiert und als Substrat in einem Fluoreszenz-Assay benutzt. Das aluminisierte $Mylar^R$ kann auf der Oberfläche eines $StiQ^R$-Probenträgers befestigt werden, der in einem $FIAX^®$-Fluorometer benutzt werden kann (IDT, Santa Clara, CA).

Im folgenden wird die Herstellung der erfindungsgemäßen network -DNA anhand von Beispielen beschrieben.

Die gereinigte network -DNA wurde durch die Kultivierung der Crithidia lucilia (ATCC, Rockville, MD) im BHI-Medium* von DIFCO erhalten. 1 l BHI wurde mit $10^6$ Organismen/ml inokuliert und im Dunkeln bei 22°C für ca. 24 h geschüttelt. Die Organismen vermehrten sich um das ca. 50 bis 100-fache während dieser Zeit, was zu einem Titer von 7 oder $8 \times 10^7$ Crithidia/ml führte. Dadurch konnte die Ernte in der späten log. Wachstumsphase der Crithidia vorgenommen werden.

Crithidia wurde durch Zentrifugation gesammelt, zweimal mit Na-EDTA Puffer gewaschen und die network -DNA entweder sofort extrahiert oder der Zentrifugationsrückstand wurde bei -70°C eingefroren.

Zur Extraktion der network -DNA wurden die Pellets der Crithidia Zellen in Na-EDTA-Puffer aus 0,15 M NaCl, 0,2M EDTA (SE-Puffer) gewaschen und zu einer maximalen Zelldichte von $1,2 \times 10^9$ Zellen/ml in SE-Puffer resuspendiert um Nuklease-Aktivität zu verhindern. Die Zellsuspension wurde durch eine 18'er stumpfe Kanüle gegeben, um Zellklumpen zu zerstören. Pronase (Calbiochem), die bei 37°C für 30 Minuten vordigeriert worden war, wurde bis zu einer Endkonzentration von 0,5 mg/ml zur Zellsuspension dazugegeben.

Eine 30 %-ige Sarkosinat-Stammlösung (N-Laurylsarkosin -Na-Salz; SIGMA) wurde bis zu einer Konzentration von 3 % zur Zellsuspension gegeben, die dann für 110 Minuten bei 60°C unter gelegentlichem Bewegen inkubiert wurde, um sämtliche Proteine und Fettbestandteile der Organismen zu verdauen. Das Zelllysat wurde danach mit einem annähernd gleichen Volumen an SE-Puffer verdünnt und 4x durch eine 18'er stumpfe Kanüle gegeben, um lineare nukleare DNA abzutrennen. Die network -DNA sind nicht empfindlich gegenüber diesem Trennprozeß, da sie klein und die Ringe sehr stabil sind.

Die network -DNA wurde danach aus dem Überstand in einer SPINCO S19 Zentrifuge bei 18.000 Umdrehungen/Minute in 60 Minute pelletiert und das Pellet wurde durch Verwirbelung in 18 ml SE-Puffer resuspendiert. Klumpen wurden durch Zerreiben entfernt. Diese Lösung wurde bei 3.000 Umdrehungen/Minute für 10 Minuten zentrifugiert und das Pellet verworfen. 18 ml Tris-EDTA-Puffer aus 0,01 M Tris-Base, pH 7,9 u. 0,001 M EDTA (TE-Puffer) wurde zugegeben und die Lösung bei 27.000 Umdrehungen/Minute für 25 Minuten in einer SW 28 Zentrifuge ($w^2t = 1,12 \times 10^{10}$) zentrifugiert. Das Pellet wurde danach in 3 ml TE-Puffer resuspendiert und solange verwirbelt bis es gelöst war.

Zur Entfernung der Proteine aus der Lysatlösung wurde eine 24 : 1 (Vol/Vol) Chloroform : Isoamylalkohol-Lösung hergestellt. 3 ml dieser Lösung wurden dem Lysat zugegeben, der Probenbehälter wurde verschlossen und durch vorsichtiges Kippen oder Schütteln des Behälters ca. 15 bis 20 Sekunden gemischt. Der Behälter wurde bei 3000 Umdrehungen/Minute 10 Minuten zentrifugiert, um die Phasentrennung zu verbessern. Die obere wässrige Phase, die network -DNA enthaltend, wurde in ein konisches Zentrifugen-Fläschen überführt und die Chloroform/Isoamylalkohol-Lösung wurde durch Zugabe von 3 ml TE-Puffer und vorbeschriebener vorsichtiger Vermischung reextrahiert. Diese Mischung wurde 10 Minuten bei 3000 Umdrehungen/Minute zentrifugiert, um die Phasentrennung zu verbessern.

Die obere, wässrige Phase wurde zur ersten wässrigen Lösung gegeben und der Extrakt bei 3000 Umdrehungen/Minute 10 Minuten in einer schwingenden Becher-Zentrifuge zentrifugiert; das Pellet wurde verworfen.

Der Extrakt wurde in zwei Milliliter Aliquots aufgeteilt und 10 ml auf -20°C vorgekühltes Ethanol zugegeben. Schließlich wurde 2 M NaCl bis zu einer Konzentration von 0.04 M NaCl zugesetzt und die Behälter wurden bei -20°C aufbewahrt bis sie benötigt wurden.

Die network -DNA kann unbegrenzt bei -20°C in dieser Form aufbewahrt und gesammelt werden um jede gewünschte Menge zu erhalten. Kontamination mit nukleärer DNA beträgt weniger als 1 %. Um die network -DNA auf eine aluminisierte $Mylar^R$ -Oberfläche aufzubringen, wurde die network -DNA durch Zentrifugation aus der Ethanollösung pelletiert und in einer Lösung aus 0.01 - 0.1 %, vorzugsweise 0.01 % kristallinem humanem Serum-Albumin in Wasser resuspendiert.

Die Konzentration der network -DNA in der Lösung wurde auf einen $OD_{260}$-Wert von 0,1 eingestellt und 50 ml dieser Lösung oder annähernd 250 ng network -DNA wurde auf jede 6,5 mm große Oberfläche gebracht. Der beladene aluminisierte $Mylar^R$ wurde bei verschiedenen Temperaturen, i.a. zwischen 2 - 37°C vollständig getrocknet.

Alternativ kann die network -DNA auch auf andere feste Substrate, wie beispielsweise Zellulose-Nitrat Filteroberflächen (SARTORIUS oder MILLIPORE) aufgebracht werden.

Auch die Methode, die network -DNA auf die Oberfläche zu applizieren, kann variiert werden, abgestimmt auf die unterschiedlichen Einsatzbereiche, beispielsweise bei der Herstellung fixierter network -DNA auf großen aluminisierten $Mylar^R$-Bögen. Die network -DNA enthaltende Lösung kann auf einen großen Bogen aluminisiertem $Mylar^R$ aufgegeben werden, die network -DNA kann sich aus der Lösung auf die aluminisierte Oberfläche absetzen, die Lösung wird dann entfernt und ersetzt durch Methanol, wodurch die network -DNA auf ihren Plätzen fixiert wird. Diese Bogen werden anschließend getrocknet und auf FIAX-$STIQs^R$ festgeklebt.

### Herstellung von Ziegen Anti-Human-Antikörper

Als Beispiel Rezeptor für den erfindungsgemäßen Assay wurden Ziegen-Anti-Human-Antikörper, die mlt Fluorescein markiert waren, kommerziell erworben (Atlantic Antibodies, Scarborough, Maine).

### Durchführung des Assays

Das Antigen-Reagenz wurde in einer 1 : 10 bis 1 : 50-fachen Verdünnung des Wirts-Serums in einem Puffer (Phosphat gepufferte Salz-Lösung pH 7,6, die 0,125 % Rinder-Serum Albumin und 0,1 % Natriumazid enthält) inkubiert. Nach 30 minütiger Inkubationszeit wurde das Antigen-Reagenz für 5 Minuten in dem Puffer gewaschen und danach zu der Rezeptorlösung, die den Ziegen-Anti-Human-Rezeptor wie oben beschrieben enthält, gegeben. Der Rezeptor war in bekannter Weise mit Fluoreszenz-Trägern markiert worden.

Anschließend wurde das Antigen-Reagenz im Puffer gewaschen und die Fluoreszenz-Intensität konnte quantitativ bestimmt werden, indem der Wert in einem geeigneten Fluorometer bestimmt wurde. Beispielsweise kann ein FIAX® Fluorometer (IDT, Santa Clara, CA) verwendet werden, um quantitativ die Fluoreszenz zu bestimmen.

Wenn die Fluoreszenzbestimmung eine positive Probe oder eine zweideutige Probe anzeigt, kann die Antigen-Reagenz-Oberfläche durch Fluoreszenz-Mikroskopie überprüft werden, um entscheiden zu können, ob die Fluoreszenz durch die network-DNA hervorgerufen wird oder unspezifischen Signalen zuzuschreiben ist, die durch ein besonderes Serum hervorgerufen wird.

### Patentansprüche

1. Verfahren zur Fluoroimmunobestimmung antinukleärer Antikörper, die gegen native DNA gerichtet sind, dadurch gekennzeichnet, daß

a) ein Antigen-Reagenz durch Aufbringung einer im wesentlichen reinen network-DNA aus Kinetoplasten auf ein festes Substrat hergestellt wird,

b) das Antigen-Reagenz in einer Serumprobe aus einem Wirt, der gegen native DNA gerichtete Antikörper produziert, komplexiert wird,

c) das komplexierte Antigen-Reagenz in einer Lösung markierter Rezeptoren, die diese Antikörper erkennen und binden, inkubiert wird, und

d) die Anwesenheit eines markierten Rezeptors, der an den Antigen-Antikörper-Komplex gebunden ist, nachgewiesen wird.

2. Verfahren gemäß Anspruch 1, dadurch gekennzeichnet, daß die Rezeptoren mit Fluoreszenzträgern markiert sind.

3. Verfahren gemäß Anspruch 2, dadurch gekennzeichnet, daß der Fluoreszenzträger Fluoreszein ist.

4. Verfahren gemäß Anspruch 1, dadurch gekennzeichnet, daß der Rezeptor ein Antikörper ist, der Antikörper aus dem Wirt erkennt und bindet.

5. Verfahren gemäß Anspruch 4, dadurch gekennzeichnet, daß der Antikörper ein Ziegen-Anti-Human-Antikörper ist.

6. Verfahren gemäß Anspruch 1, dadurch gekennzeichnet, daß das komplexierte Antigen-Reagenz vor der Inkubation in der Lösung markierter Rezeptoren gewaschen wird.

7. Verfahren gemäß Anspruch 6, dadurch gekennzeichnet, daß das komplexierte Antigen-Reagenz vor dem Nachweis des markierten Rezeptors in einem neutralen Puffer gewaschen wird.

8. Verfahren gemäß Anspruch 1, dadurch gekennzeichnet, daß das feste Substrat aluminisiertes Mylar[R] ist.

9. Im wesentlichen reine network-DNA aus Kinetoplasten, die an ein festes Substrat gebunden ist.

10. Assay-Kit zur Fluoroimmunobestimmung antinukleärer Antikörper, die gegen native DNA gerichtet sind, dadurch gekennzeichnet, daß er

a) eine an einem festen Subrat gebundene, im wesentlichen reine network-DNA aus Kinetoplasten,

b) markierte Rezeptoren, die die nachzuweisenden Antikörper erkennen und binden, und

c) Wasch- und Pufferlösungen enthält.

11. Assay-Kit gemäß Anspruch 10, dadurch gekennzeichnet. daß das feste Substrat aluminisiertes Mylar[R] ist.

12. Assay-Kit gemäß Anspruch 10 oder 11, dadurch gekennzeichnet, daß die Rezeptoren mit Fluoreszenzträgern, beispielsweise mit Fluoreszein, markiert sind.

13. Assay-Kit gemäß Anspruch 10, 11 oder 12, dadurch gekennzeichnet, daß die Rezeptoren Antikörper, beispielsweise Ziegen-Anti-Human-Antikörper, sind.

### Claims

1. A method for fluoroimmunoassay of antinuclear antibodies against native DNA characterized in that

a. an antigen reagent is prepared by applying substantially pure network DNA comprising kinetoplasts to a solid substrate,

b. complexing the antigen reagent in a serum solution derived from a host which produces antibodies to native DNA,

c. incubating the complexed antigen reagent in a solution of labeled receptors capable of recognizing and binding these antibodies, and

d. evidencing the presence of a labeled receptor bound to the antigen-antibody complex.

2. A method according to claim 1, characterized in that the receptors are labeled with fluorescent carriers.

3. A method according to claim 2, characterized in that the fluorescent carrier is fluorescein.

4. A method according to claim 1, characterized in that the receptor is an antibody capable of recognizing and binding antibodies derived from the host.

5. A method according to claim 4, characterized in that the antibody is Goat anti-Human antibody.

6. A method according to claim 1, characterized in that the complexed antigen reagent is washed prior to incubating it in the solution of labeled receptors.

7. A method according to claim 6, characterized in that the complexed antigen reagent is washed in a neutral buffer prior to analyzing the labeled receptor.

8. A method according to claim 1, characterized in that the solid substrate is aluminized Mylar$^R$.

9. Substantially pure network DNA from kinetoplasts which has been bound to a solid substrate.

10. Assay-kit for fluoroimmunoassay of antinuclear antibodies directed against native DNA characterized by comprising

a. a substantially pure network DNA from kinetoplasts bound to a solid substrate,

b. labeled receptors which recognize and bind the antibodies to be recognized, and

c. washing and buffering solutions.

11. Assay-kit according to claim 10, characterized in that the solid substrate is aluminized Mylar$^R$.

12. Assay-kit according to claim 10 or 11, characterized in that the receptors have been labeled by fluorescence carriers, e.g. fluorescein.

13. Assay-kit according to claim 10, 11 or 12, characterized in that the receptors are antibodies, e.g. goat anti-human antibodies.

## Revendications

1. Procédé pour la fluoroimmunodétermination détermination d'anticorps antinucléaires, caractérisé en ce que:

a) un réactif antigène est fabriqué par application d'un réseau principalement pur de DNA provenant de cinétoplastes sur un substrat solide,

b) en ce que le réactif antigène est complexé dans un échantillon de sérum provenant d'un hôte dont il est supposé qu'il forme des anticorps contre la DNA native,

c) le complexe réactif-antigène est incubé dans une solution de récepteurs marqués reconnaissant et combinant ces anticorps,

d) la présence d'un récepteur marqué combiné au complexe antigène-anticorps est décelée.

2. Procédé selon la revendication 1, caractérisé en ce que les récepteurs sont marqués par des porteurs de fluorescence.

3. Procédé selon la revendication 2, caractérisé en ce que le porteur de fluorescence est la fluorescéine.

4. Procédé selon la revendication 1, caractérisé en ce que le récepteur est un anticorps reconnaissant et combinant des anticorps provenant de l'hôte.

5. Procédé selon la revendication 4, caractérisé en ce que l'anticorps est anticorps chèvre-anti-humain.

6. Procédé selon la revendication 1, caractérisé en ce que le réactif antigène complexé est lavé avant l'incubation dans la solution de récepteurs marqués.

7. Procédé selon la revendication 6, caractérisé en ce que le réactif antigène complexé est lavé dans un tampon neutre avant de déceler le récepteur marqué.

8. Procédé selon la revendication 1, caractérisé en ce que le substrat solide est le Mylar® aluminisé.

9. Principalement réseaux DNA purs de cinétoplastes combinés à un substrat solide.

10. Nécessaire à essai pour fluoroimmunodétermination d'anticorps antinucléaires, orienté contre la DNA native, caractérisé en ce qu'il contient

a) un réseau DNA principalement pur de cinétoplastes, combiné à un substrat solide,

b) des récepteurs marqués reconnaissant et combinant les anticorps à déceler, et

c) des solutions de lavage et des solutions tampons.

11. Nécessaire à essai selon la revendication 10, caractérisé en ce que ie substrat solide est du Mylar® aluminisé.

12. Nécessaire à essai selon la revendication 10 ou 11, caractérisé en ce que les récepteurs sont marqués par des porteurs de fluorescence par exemple à la fluorescéine.

13. Nécessaire à essai selon la revendication 10, 11 ou 12, caractérisé en ce que les récepteurs sont des anticorps, par exemple des anticorps chèvre-anti-humain.